# EUROPÄISCHE PATENTANMELDUNG

(11) **EP 4 667 449 A1**
(43) Veröffentlichungstag der Anmeldung: **24.12.2025**
(21) Anmeldenummer: 24020202.8
(22) Anmeldetag: 18.06.2024
(51) Int. Cl.: C07C 29/151, C07C 31/04

(54) **VERFAHREN UND ANLAGE ZUR HERSTELLUNG VON METHANOL**

(71) Anmelder: Linde GmbH, 82049 Pullach (DE)
(72) Erfinder: Dillig, Marius, 82049 Pullach (DE); Fischer, Anna-Maria, 82049 Pullach (DE); Korn, Wibke, 82049 Pullach (DE)
(74) Vertreter: Fischer, Werner

(57) **Zusammenfassung**

Es wird ein Verfahren (100, 200) zur Herstellung von Methanol vorgeschlagen, das das Reinigen eines Rohkohlendioxidstroms (103) unter Erhalt eines Reinkohlendioxidstroms (104), das Einspeisen des Reinkohlendioxidstroms (104) in einen Methanolsynthesekreislauf sowie das Ausschleusen eines Rohmethanolstroms (111) aus dem Methanolsynthesekreislauf umfasst, wobei das Reinigen des Rohkohlendioxidstroms (103) absorptiv und unter Verwendung des Rohmethanolstroms (111) oder eines Anteils (111a) hiervon als Absorptionsflüssigkeit vorgenommen wird. Eine entsprechende Anlage wird ebenfalls vorgeschlagen.

## Beschreibung

### Gebiet

Die vorliegende Offenbarung betrifft ein Verfahren zur Herstellung von Methanol und eine entsprechende Anlage.

### Hintergrund

Bisher basiert die Herstellung von Methanol und Derivaten wie Dimethylether meist auf der Reaktion von Synthesegas, das Kohlenmonoxid und Wasserstoff enthält, in einem sogenannten Methanolsynthesekreislauf. Das Synthesegas, ein Gemisch aus Wasserstoff und Kohlestoffoxiden, wird dabei in herkömmlichen Verfahren typischerweise aus durch Dampfreformierung oder partiellen Oxidation aus fossilen kohlenstoffhaltigen Rohstoffen wie Erdgas oder Kohle unter Freisetzung von Kohlendioxid gewonnen.

Um das Ausmaß des globalen Klimawandels durch Kohlendioxidemissionen zu begrenzen, wurde die Nutzung von Kohlendioxid als Kohlenstoffquelle für die Methanolproduktion vorgeschlagen. Das Kohlendioxid kann dabei mit Wasserstoff, der beispielsweise durch die Elektrolyse von Wasser erhalten wird, zum Synthesegas gemischt werden.

### Übersicht

Vor diesem Hintergrund werden ein Verfahren zur Herstellung von Methanol und eine entsprechende Anlage mit den Merkmalen der unabhängigen Patentansprüche vorgeschlagen. Ausgestaltungen sind Gegenstand der abhängigen Patentansprüche sowie der nachfolgenden Beschreibung.

Das vorgeschlagene Verfahren zur Herstellung von Methanol umfasst das Reinigen eines Rohkohlendioxidstroms unter Erhalt eines Reinkohlendioxidstroms, das Einspeisen des Reinkohlendioxidstroms in einen Methanolsynthesekreislauf und das Ausschleusen eines Rohmethanolstroms aus dem Methanolsynthesekreislauf, wobei das Reinigen des Rohkohlendioxidstroms absorptiv und unter Verwendung des Rohmethanolstroms oder eines Anteils hiervon als Absorptionsflüssigkeit vorgenommen wird.

In dem hier vorgeschlagenen Verfahren erfolgt also die Reinigung des Rohkohlendioxidstroms insbesondere bezüglich bestimmter, bei der Methanolsynthese nachteiliger Komponenten mit Rohmethanol und insbesondere in einer Waschkolonne. Das Rohmethanol besitzt im Vergleich etwa zu Wasser eine erhöhte Aufnahmefähigkeit für solche Stoffe. Das bei der Wäsche mit den aus dem Rohkohlendioxidstrom abgetrennten Komponenten beladene Rohmethanol wird einer Methanolaufbereitung zugeführt, wobei es in eine sogenannte Toppingkolonne gefahren wird, um gelöste Gase und leichtsiedende Komponenten zu entfernt und als sogenannte Light Ends bzw. als Purgegas abzuziehen, die beispielsweise als Brenngas genutzt werden. Weitere Vorteile sind im Zusammenhang mit Ausführungsformen weiter unten erläutert.

In bestimmten Ausgestaltungen des vorgeschlagenen Verfahrens ist vorgesehen, dass die zum Reinigen des Rohkohlendioxidstroms eingesetzte Waschkolonne bei einem Druck zwischen 5 bis 35 bar betrieben wird. Möglich ist es aber auch, die Reinigung bei einem höheren Druck durchzuführen, der beispielsweise zwischen 50 und 100 bar liegt und dem Druck entspricht, mit dem der Methanolsynthesekreislauf betrieben wird. Diese Verfahrensvariante hat den Vorteil, dass der so erhaltene Reinkohlendioxidstrom ohne weiteren Verdichtungsaufwand in den Methanolsynthesekreislauf eingespeist werden kann.

In bestimmten Ausgestaltungen des vorgeschlagenen Verfahrens ist vorgesehen, dass das Rohmethanol einen aus Wasser und Methanol gebildeten Anteil enthält, der seinerseits 45 bis 95 mol-% Methanol und ansonsten Wasser umfasst. Der Methanol- und Wassergehalt ergibt sich aus der Zusammensetzung des Einsatzes, insbesondere durch das Verhältnis von Kohlendioxid zu Kohlenmonoxid. Es versteht sich, dass das Rohmethanol auch einen nicht durch Wasser und Methanol gebildeten Anteil aufweisen kann, der bestimmte Nebenkomponenten umfasst. Ein derartiges Rohmethanol weist eine besonders gute Aufnahmefähigkeit für die aus dem Rohkohlendioxidstrom abzutrennenden Komponenten auf.

In bestimmten Ausgestaltungen des vorgeschlagenen Verfahrens ist vorgesehen, dass der für das Reinigen des Rohkohlendioxidstroms verwendete Anteil des Rohmethanolstroms gemeinsam mit den nicht für die Reinigung eingesetzten Anteil des Rohmethanols einer fachüblich zum Erhalt von Reinmethanol eingesetzten und aus dem Stand der Technik bekannten Methanolaufbereitung zugeführt wird. Hier können die aus dem Rohkohlendioxidstrom aufgenommenen Komponenten ohne Zusatzaufwand wieder entfernt werden.

In bestimmten Ausgestaltungen des vorgeschlagenen Verfahrens ist vorgesehen, dass die Methanolaufbereitung unter Verwendung wenigstens zweier Aufreinigungskolonnen durchgeführt wird, von denen die erste zum Entfernen von leichteren Komponenten, darunter solchen, wie sie aus dem Rohkohlendioxid aufgenommen wurden, zum Einsatz kommt. Es können jedoch grundsätzlich alle zur Aufreinigung von Rohmethanol zu Reinmethanol bekannten Verfahren verwendet werden.

In bestimmten Ausgestaltungen des vorgeschlagenen Verfahrens ist vorgesehen, dass für das Reinigen des Rohkohlendioxidstroms ein erster Anteil des Rohmethanolstroms verwendet wird und aus einem zweiten Anteil des Rohmethanolstroms Kohlendioxid unter Verwendung von Wasserstoff ausgetrieben wird, wobei das ausgetriebene Kohlendioxid oder ein Teil hiervon in den Methanolsynthesekreislauf zurückgeführt wird.

In bestimmten Ausgestaltungen des vorgeschlagenen Verfahrens ist vorgesehen, dass der Methanolsynthesekreislauf einen Methanolsynthesereaktor umfasst, dem ein Methanol, Wasser und Kohlendioxid enthaltender Produktstrom entnommen wird. Es versteht sich, dass der Produktstrom auch beispielsweise Kohlenmonoxid und weitere Komponenten wie Inerte und Nebenprodukte der Methanolsynthese umfasst. Hierbei ist vorgesehen, dass der Produktstrom unter Erhalt des Rohmethanolstroms und eines Rückführstroms einer Teilkondensation unterworfen wird. Der Methanolsynthesekreislauf wird auf dem erwähnten Druckniveau betrieben und kann in an sich bekannter Weise aufgebaut sein.

In bestimmten Ausgestaltungen des vorgeschlagenen Verfahrens ist vorgesehen, dass der Methanolsynthesereaktor als Isothermalreaktor ausgebildet ist, der ein Katalysatorbett aufweist, in dem gewickelte Dampferzeugungsrohre angeordnet sind, wobei die Dampferzeugungsrohre mit Kesselspeisewasser beschickt werden, und wobei mittels der Dampferzeugungsrohre Dampf, insbesondere Sattdampf, erzeugt wird. Der Methanolsynthesereaktor kann dabei insbesondere als sogenannter Linde-Isothermalreaktor ausgebildet sein, wie er in der Fachliteratur beschrieben ist.

Wie beispielsweise bei Lembeck, M., "Linde isothermal reactor for methanol synthesis", Linde Berichte aus Technik und Wissenschaft, Band 58, Seiten 5 bis 8, dargestellt, weist ein derartiger Reaktor eine Anordnung mit gewickelten, in einem Festbettkatalysator untergebrachten Dampferzeugungsrohren auf. Das durch das Innere dieser Rohre geführte Speisewasser wird zu Mitteldruckdampf umgesetzt und nimmt dabei die bei der exothermen Methanolsynthese freigesetzte Reaktionswärme auf, wodurch eine isotherme Verfahrensführung ermöglicht wird.

In bestimmten Ausgestaltungen des vorgeschlagenen Verfahrens ist vorgesehen, dass der Rückführstrom oder ein Teil hiervon mit Kohlendioxid des Reinkohlendioxidstroms und Wasserstoff vereinigt, verdichtet, erwärmt und dem Methanolsynthesereaktor erneut zugeführt wird. Auf diese Weise wird der Synthesekreislauf geschlossen.

In bestimmten Ausgestaltungen des vorgeschlagenen Verfahrens ist vorgesehen, dass der Methanolsynthesereaktor unter Verwendung eines Kupfer, Zinkoxid und Aluminiumoxid enthaltenden Katalysators betrieben wird. Entsprechende Katalysatoren profitieren von der Kohlendioxidreinigung in besonderer Weise.

In bestimmten Ausgestaltungen des vorgeschlagenen Verfahrens ist vorgesehen, dass das Reinigen des Rohkohlendioxidstroms derart durchgeführt wird, dass ein Gehalt des Reinkohlendioxidstroms an zumindest einer schwefelhaltigen Verbindung und/oder zumindest einer stickstoffhaltigen Verbindung und/oder zumindest einer Halogenverbindung und/oder von Cyanwasserstoff unterhalb eines vorgegebenen Schwellwerts liegt. Hierbei kann insbesondere eine Anpassung des für das Reinigen des Rohkohlendioxidstroms verwendeten Anteils des Rohmethanolstroms erfolgen.

In bestimmten Ausgestaltungen des vorgeschlagenen Verfahrens ist vorgesehen, dass sauerstoffhaltiger Rohwasserstoff unter Verwendung einer katalytischen Reinigungseinheit einer Sauerstoffentfernung unterworfen wird, um Wasserstoff für die Einleitung in den Methanolsynthesekreislauf zu erhalten.

Die vorgeschlagene Anlage zur Herstellung von Methanol ist zum Reinigen eines Rohkohlendioxidstroms unter Erhalt eines Reinkohlendioxidstroms, zum Einspeisen des Reinkohlendioxidstroms in einen Methanolsynthesekreislauf, und zum Ausschleusen eines Rohmethanolstroms aus dem Methanolsynthesekreislauf eingerichtet, wobei das Reinigen des Rohkohlendioxidstroms absorptiv und unter Verwendung des Rohmethanolstroms oder eines Anteils hiervon als Absorptionsflüssigkeit vorgenommen wird.

Zu weiteren Merkmalen und Vorteilen einer entsprechenden Anlage und Ausgestaltungen hiervon sei auf die obigen Erläuterungen betreffend das erfindungsgemäß vorgeschlagene Verfahren und seine Ausgestaltungen ausdrücklich verwiesen, da diese hierfür in gleicher Weise gelten.

Entsprechendes gilt auch für eine Anlage, die gemäß einer Ausgestaltung der Erfindung dazu eingerichtet ist, ein Verfahren gemäß einer beliebigen Ausgestaltung der vorliegenden Erfindung durchzuführen.

### Zeichnungen

Ausführungsformen von hier vorgeschlagenen Lösungen werden nachfolgend rein beispielhaft unter Bezugnahme auf die beigefügte Zeichnung beschrieben, wobei
Figur 1 ein Vergleichsverfahren veranschaulicht;
Figur 2 ein Verfahren gemäß einer Ausgestaltung veranschaulicht; und
Figur 3 ein Verfahren gemäß einer Ausgestaltung veranschaulicht.

### Ausführungsformen

Die nachfolgend beschriebenen Ausführungsformen und Ausgestaltungen werden lediglich zu dem Zweck beschrieben, den Leser beim Verständnis der beanspruchten und zuvor erläuterten Merkmale zu unterstützen. Sie stellen lediglich repräsentative Beispiele dar und sollen hinsichtlich der Merkmale vorgeschlagener Verfahren und Vorrichtungen nicht abschließend und/oder beschränkend betrachtet werden.

Sämtliche hier verwendeten Prozentangaben können sich auf Mol-, Mengen- oder Volumenanteile beziehen. Druckangaben in bar sind, soweit nicht anders erläutert, insbesondere als Absolutdrücke zu verstehen.

Ist vorliegend von einem "Teil" eines Stoffstroms die Rede, kann es sich hierbei um einen Mengenanteil mit gleicher Zusammensetzung handeln, der von einem Ausgangsstrom lediglich abgezweigt wurde, aber auch um einen Anteil abweichender Zusammensetzung und ggf. nur eine Komponente des Ausgangsstroms, der mittels eines Verfahrens wie Kondensation, Evaporation, Sieden, Destillieren, Rektifizieren, Absorbieren, Adsorbieren, Flashen, Membrantrennen, Abscheiden oder dergleichen gebildet wird oder bei einem entsprechenden Schritt als Rest verbleibt. Ein "Teil" kann auch nach einer Kombination beliebiger der vorstehend benannten Schritte vorliegen, beispielsweise nach trenntechnischer Bearbeitung abgezweigten Anteils.

Für die Methanolproduktion aus Kohlendioxid lassen sich im Wesentlichen zwei Hauptwege unterscheiden:
Der erste Weg kann als direkte Hydrierung von Kohlendioxid beschrieben werden, wobei Kohlendioxid zusammen mit einer wasserstoffhaltigen Coeinspeisung dem Methanolsynthesekreislauf zugeführt wird. Die Bruttoreaktion, die in einem solchen Kreislauf stattfindet, kann mit der nachfolgend angegeben Reaktionsgleichung (1) beschrieben werden.

CO₂ + 3 H₂ --+ CH₃OH + H₂O (1)

Der zweite Weg umfasst eine Kohlendioxidelektrolyse oder eine Coelektrolyse, mittels derer zumindest ein Teil des Kohlendioxideinsatzes zu Kohlenmonoxid umgewandelt wird, welches dann seinerseits einem Kreislauf zugeführt wird, der dem herkömmlichen Methanolsynthesekreislauf ähnelt.

Die Bruttoreaktion bei der elektrochemischen Umwandlung von Kohlendioxid in Kohlenmonoxid kann mit der nachfolgend angegebenen Reaktionsgleichung (2) beschrieben werden.

CO + 2 H₂ --+ CH₃OH (2)

Unabhängig davon, ob die direkte Hydrierung von Kohlendioxid gemäß Reaktionsgleichung (1) oder ein auf Kohlenmonoxid basierendes Verfahren gemäß Reaktionsgleichung (2) verwendet wird, kann die allgemeine Ausgestaltung eines entsprechenden Kreislaufs wie folgt beschrieben werden, wobei hier vorgeschlagene Ausgestaltungen die Variante gemäß Reaktionsgleichung (1) betreffen.

Die jeweiligen Einsatzströme werden zusammengeführt und auf einen Druck von typischerweise 50-100 bar verdichtet. Je nach Herkunft der Einsatzstoffe können die verschiedenen Einsatzströme dabei separat oder nach der Zusammenführung verdichtet werden. Ein entsprechend gebildeter Gesamteinsatzstrom wird mit einem Rezyklatstrom vereinigt, vorgewärmt, und in einem oder mehreren Katalysatorbetten zur Reaktion gebracht. Der gebildete Produktstrom wird abgekühlt, die Hauptreaktionsprodukte werden zumindest teilweise kondensiert und aus dem Kreislauf entfernt, und die gasförmigen Reaktanten werden zumindest teilweise in den erwähnten Rezyklatstrom überführt. Die flüssigen Reaktionsprodukte werden aufgereinigt, typischerweise in einer oder mehreren Destillationskolonnen.

Das in entsprechenden Verfahren verwendete Kohlendioxid kann aus unterschiedlichen Quellen stammen. Beispielsweise kann eine Abtrennung aus Rauchgasen, Synthesegasen oder Gasen biogenen Ursprungs erfolgen. Das Kohlendioxid kann daher verschiedenste Verunreinigungen enthalten. Wie erwähnt, kann der Wasserstoff aus beliebigen Quellen stammen, insbesondere aber aus einer Wasserelektrolyse beliebiger Art.

Wasserstoff und Kohlendioxid können also nach einer geeigneten Reinigung gemischt und als Makeupgas dem Methanolsynthesekreislauf stromauf eines mit einem geeigneten Katalysator ausgestatteten Synthesereaktor zugeführt werden. Der Katalysator kann dabei insbesondere die Komponenten Kupfer, Zinkoxid und Aluminiumoxid aufweisen (Cu-ZnO-Al₂O₃).

Ein Teil der dem Synthesereaktor zugeführten Komponenten reagieren im Synthesereaktor zu Methanol. Durch Kühlung eines Komponentengemischs am Reaktoraustritt können die kondensierbaren Bestandteile als Rohmethanol abgeschieden werden. Das flüssige Rohmethanol umfasst überwiegend Methanol und Wasser sowie in geringen Konzentrationen Nebenprodukte der Methanolsynthese wie höhere Alkohole, Dimethylether, Methylformate und Ketone sowie in geringen Mengen gelöste Gase, vor allem Kohlendioxid, Kohlenmonoxid und Wasserstoff.

Nicht abreagierter Wasserstoff und nicht abreagiertes Kohlendioxid sowie gebildetes Kohlenmonoxid bleiben hauptsächlich in der Gasphase, werden in einem Abscheider abgeschieden, mit Makeupgas gemischt und wieder zum Synthesereaktor zurückgeführt. Das Rohmethanol wird anschließend einer Aufreinigung zugeführt, in der Leichtsieder sowie Schwersieder und Wasser entfernt werden. Diese Aufreinigung erfolgt typischerweise in einem ein- oder mehrstufigen Destillationsprozess.

Die erwähnten Katalysatoren, die auch weitere Komponenten aufweisen können, zeichnen sich dadurch aus, dass eine Reihe von Verbindungen bei Überschreitung von bestimmten Konzentrationen als Katalysatorgifte wirken können. Katalysatorgifte führen dabei insbesondere zu einer rascheren Deaktivierung des Katalysators. Die zulässigen Konzentrationen entsprechender Verbindungen unterscheiden sich zwischen den Katalysatorherstellern und sind den jeweiligen Spezifikationen zu entnehmen. Ein Makeupgas kann beispielsweise Wasser, Sauerstoff, Wasserstoff, Kohlenmonoxid Ethylen und ungesättigte Kohlenwasserstoffe enthalten, sowie außerdem Schwefelverbindungen wie Schwefelwasserstoff, Schwefeloxide und organische Schwefelspezies, Chlor, Halogene, halogenierte Verbindungen, Cyanwasserstoff, Stickstoffoxide, Ammoniak, Amine, Alkalimetalle, Siliciumspezies und Schwermetalle, wobei zumindest einige dieser Verbindungen Katalysatorgifte darstellen können.

Unter anderem adsorbieren schwefelhaltige Verbindungen an der Kupferoberfläche und blockieren diese. Daher müssen diese auf sehr niedrige Konzentrationen entfernt werden (typischerweise weniger als 0,1 ppmv, d.h. Millionstel Anteile auf Volumenbasis). Stickstoffhaltige Verbindungen (Ammoniak, Amine, Stickstoffoxide) wirken einerseits ebenfalls als Katalysatorgifte, zum zweiten bildet sich bei Vorhandensein dieser Verbindungen am Katalysator Trimetylamin, was die Methanolqualität aufgrund des starken Geruchs negativ beeinflusst. Cyanwasserstoff wird teilweise, abhängig vom eingesetzten Typ des Katalysators, als unkritisch für den Synthesekatalysator betrachtet. Dennoch wird Cyanwasserstoff von einigen Katalysatorherstellern als Katalysatorgift mit niedriger Spezifikation eingestuft (frei bzw. weniger als 0,1 ppmv). Der eingesetzte Wasserstoff, ist meist sehr rein, lediglich Sauerstoff (und ggf. Kaliumhydroxid aufgrund der alkalischen Elektrolyse) müssen entfernt werden, was für Sauerstoff in einem der Elektrolyse nachgeschalten Reinigungsschritt durch katalytische Umsetzung erfolgt (sogenannter DeOxo-Reaktor).

Im eingesetzten Kohlendioxid können, je nach Quelle und Abtrennverfahren, einzelne oder eine Kombination der oben genannten Verunreinigungen enthalten sein, die ein Reinigungsverfahren notwendig machen.

Typische Reinigungsverfahren, mittels derer die oben genannten Komponenten entfernt werden können, sind in der nachfolgenden Tabelle 2 angegeben, sollen die vorliegende Erfindung aber in keiner Weise einschränken:

**Tabelle 2**

| Verbindungen | Einsatz | Entfernung |
|---|---|---|
| Sauerstoff | Wasserstoff, Kohlendioxid | katalytisch (DeOxo) |
| Schwefelwasserstoff | Kohlendioxid | Aminwäsche, Adsorption, z.B. an ZnO-Bett |
| Schwefeloxide | Kohlendioxid | Wasser-/Laugewäsche, ggf. mit nachfolgendem adsorptivem oder kryogenem Abtrennschritt |
| organischer Schwefel | Kohlendioxid | Hydrierung mit nachfolgender Schwefelwasserstoffentfernung oder katalytische Oxidation mit anschließender Schwefeloxidentfernung und katalytischer Sauerstoffentfernung |
| Carbonylsulfid | Kohlendioxid | Aminwäsche, Hydrolyse und Adsorption an ZnO-Bett |
| Ammoniak | Kohlendioxid | Wasser-/Laugewäsche |
| Amine | Kohlendioxid | Wasser-/Laugewäsche |
| Stickstoffoxide | Kohlendioxid | selektive katalytische Reduktion |
| Cyanwasserstoff | Kohlendioxid | Laugewäsche |
| Chlorwasserstoff | Kohlendioxid | Adsorption an Zinkoxidbett |
| höhere Kohlenwasserstoffe | Kohlendioxid | Katalytisch |

Hier vorgeschlagene Ausgestaltungen beruhen auf der Erkenntnis, dass die Reinigung des Kohlendioxids vor allem bezüglich Schwefelkomponenten (Schwefeloxide, Schwefelwasserstoff, organischer Schwefel wie Mercaptane und Thiophene), Ammoniak, Aminen, Stickstoffoxide, Cyanwasserstoff und höheren Kohlenwasserstoffen mit dem Rohmethanol stromab eines Hochdruckabscheiders in einer Waschkolonne für Kohlendioxid erfolgen kann. Das Methanol besitzt eine erhöhte Aufnahmefähigkeit für diese Stoffe gegenüber einer Wasserwäsche.

Das Rohmethanol wird anschließend, wie im Standardprozess, in die sogenannte Toppingkolonne gefahren, wo gelöste Gase und leichtsiedende Komponenten wieder entfernt werden und als sogenannte Light Ends bzw. Purgegas, beispielsweise als Brenngas, verwendet werden können.

Das Kohlendioxid kann insbesondere bei erhöhtem Druck (etwa 5 bis 30 bar) in Kontakt mit dem Rohmethanolabscheider gebracht werden. Dies kann in einer Waschkolonnen, die je nach benötigter Waschmittelmenge als Packungskolonne oder als Bodenkolonne ausgeführt ist, erfolgen, wobei teils nur wenige theoretische Trennstufen (beispielsweise 4 bis 15 Trennstufen) erforderlich sind.

Besonders vorteilhaft ist, dass das Rohmethanol eine erhöhte Löslichkeit gegenüber den Zielverunreinigungen aufweist und diese damit besser als in einer Wasserwäsche entfernt werden können. Diese Löslichkeit ist z.B. für die Komponenten Schwefelwasserstoff um Faktor 10 und für Schwefeloxide um Faktor 7 erhöht. Die nachfolgende Tabelle 3 gibt eine Übersicht über die angenommenen Konzentrationen und die Erhöhung der Löslichkeit, jeweils bei 298 K, wobei es sich bei den angenommenen Konzentrationen und Zielwerten lediglich um angenommene Konzentrationen in einem beispielhaften Einsatzstrom bzw. eine angenommene maximale Konzentration in einem Makeupgas in den Methanolsynthesekreislauf handelt. Die entsprechenden Werte schränken die Erfindung nicht ein. Die letzte Spalte gibt die Löslichkeit in Methanol in Bezug auf die mit 1 angenommene Löslichkeit in Wasser als gerundenen Faktor an.

**Tabelle 3**

| Komponente | Einsatz | Konzentration | Zielwert | Löslichkeit in Methanol |
|---|---|---|---|---|
| | | ppmv | ppmv | Faktor bezogen auf Wasser |
| Sauerstoff | Wasserstoff, Kohlendioxid | 100 | 10 | 7 |
| Schwefelwasserstoff | Kohlendioxid | 10 | 0,1 | 10 |
| Schwefeloxide | Kohlendioxid | 10 | 0,1 | 7 |
| Ammoniak | Kohlendioxid | 10 | 1 | 0,1 |
| Amine | Kohlendioxid | 10 | 1 | sehr hoch |
| Stickstoffoxide | Kohlendioxid | 10 | 1 | sehr niedrig |
| Cyanwasserstoff | Kohlendioxid | 10 | 1 | 4 |
| Schwefelwasserstoff | Kohlendioxid | 1 | 0,05 | 15 |

Anhand der Löslichkeiten und Simulationen lassen sich Schwefeloxide, organischer Schwefel, Ammoniak, Amine, Cyanwasserstoff, Chlorwasserstoff und andere Halogenwasserstoffe sowie höhere Kohlenwasserstoffe in den hier vorgeschlagenen Ausgestaltungen besonders gut entfernen. Schwefelwasserstoff und Carbonylsulfid lassen sich begrenzt gut entfernen, und zwar insbesondere bei Verwendung einer erhöhten Rohmethanolmenge als Waschmittel. Dies hat ggf. erhöhte Kohlendioxidverluste zur Folge. Nicht entfernen lassen sich in den vorgeschlagenen Ausgestaltungen Stickstoffoxide und Sauerstoff.

Ein weiterer Vorteil des ist die Strippwirkung des Kohlendioxids auf die (geringe) Wasserstoffmenge, die im Rohmethanol gelöst ist. Diese kann desorbiert werden und steht für die Methanolsynthese wieder zur Verfügung. Das Rohmethanol ist im Rohmethanolabscheider bereits mit Kohlendioxid bei erhöhtem Druck (beispielsweise 50 bis 100 bar Absolutdruck) gesättigt, so dass, je nach Druck, weniger oder kein weiteres Kohlendioxid im Rohmethanol in Lösung geht.

Beide Aspekte ergeben sich insbesondere aus den nachfolgenden Tabellen 4a und 4b, die Kohlendioxid- und Methanolverluste bei einem beispielhaften Methanolprozess für Drücke von 30 bar (Tabelle 4a) und 10 bar (Tabelle 4b) in der Wäsche bzw. Waschkolonneangibt. Mit Nm³/h sind jeweils Normkubikmeter pro Stunde angegeben.

**Tabelle 4a**

| Zufuhr Kohlendioxid | Produziertes Rohmethanol | Rohmethanol zur Wäsche | | Abfuhr Kohlendioxid | Verlust Kohlendioxid | | Rückgewinnung Wasserstoff |
|---|---|---|---|---|---|---|---|
| [Nm³/h] | [kg/h] | [kg/h] | [%] | [Nm³/h] | [Nm³/h] | [%] | [Nm³/h] |
| 10000 | 20000 | 20000 | 100% | 9189 | 811 | 8,1% | 176 |
| 10000 | 20000 | 10000 | 50% | 9565 | 435 | 4,4% | 88 |
| 10000 | 20000 | 5000 | 25% | 9767 | 233 | 2,3% | 44 |
| 10000 | 20000 | 2000 | 10% | 9911 | 89 | 0,9% | 18 |

**Tabelle 4b**

| Zufuhr Kohlendioxid | Produziertes Rohmethanol | Rohmethanol zur Wäsche | | Abfuhr Kohlendioxid | Verlust Kohlendioxid | | Rückgewinnung Wasserstoff |
|---|---|---|---|---|---|---|---|
| [Nm3/h] | [kg/h] | [kg/h] | [%] | [Nm3/h] | [Nm3/h] | [%] | [Nm3/h] |
| 10000 | 20000 | 20000 | 100% | 9851 | 149 | 1,5% | 176 |
| 10000 | 20000 | 10000 | 50% | 9910 | 90 | 0,9% | 88 |
| 10000 | 20000 | 5000 | 25% | 9952 | 48 | 0,5% | 44 |
| 10000 | 20000 | 2000 | 10% | 9984 | 16 | 0,2% | 18 |

Das gewaschene Kohlendioxid kann nach der Waschkolonne dem Wasserstoff zugegeben und kann auf Synthesedruck gebracht werden.

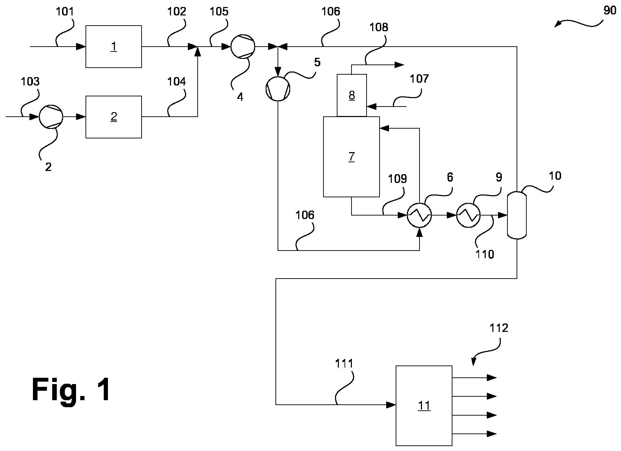

In Figur 1 ist ein Vergleichsverfahren dargestellt und insgesamt mit 90 bezeichnet.

Dem Vergleichsverfahren 90 wird ein Wasserstoffstrom 101 zugeführt, der beispielsweise aus einer nicht dargestellten Elektrolyse stammen kann und Restmengen an Sauerstoff enthalten kann. Zur Entfernung des Sauerstoffs kann eine katalytische Sauerstoffentfernungseinheit 1 vorgesehen sein, der ein entsprechend aufgereinigter Wasserstoffstrom 102 entnommen wird.

Dem Vergleichsverfahren 90 wird ferner ein Rohkohlendioxidstrom 103 zugeführt, der in einem Kohlendioxidverdichter 2 verdichtet und in einer Kohlendioxidreinigungseinheit 3 aufgereinigt wird. Der Kohlendioxidreinigungseinheit 3 wird ein Reinkohlendioxidstrom 104 entnommen. Der aufgereinigte Wasserstoffstrom 102 und der Reinkohlendioxidstrom 104 werden zu einem Frischeinsatzstrom 105 vereinigt, welcher in einem Frischeinsatzverdichter 4 verdichtet und anschließend in den eigentlichen Methanolsynthesekreislauf eingespeist werden kann.

Der Methanolsynthesekreislauf umfasst einen Rückführstrom 106, der nach Vereinigung mit dem Frischeinsatzstrom 105 als Sammelstrom 106 in einem Rückführverdichter 5 verdichtet und anschließend in einem Feed-Effluent-Wärmetauscher 6 erwärmt und in einen Methanolsynthesereaktor 7 eingespeist wird. Dem Methanolsynthesereaktor 7 ist eine Dampferzeugungseinheit 8 zugeordnet, die mit Kesselspeisewasser 107 gespeist und der Sattdampf 108 entnommen wird. Dies ist für den Gegenstand der vorliegenden Erfindung jedoch nicht entscheidend

Dem Methanolsynthesereaktor 7 wird ein Produktstrom 109 entnommen, welcher in dem Feed-Effluent-Wärmetauscher 6 und anschließend einem Wasserkühler 9 gekühlt und dabei teilkondensiert wird. Es können hier auch weitere Kühlstufen zwischengeschaltet sein, beispielsweise eine Wärmeintegration zu einem Reboiler einer Destilaltionskolonne, ein Luftkühler oder andere Formen der Wärmeauskopplung. Ein hierbei erhaltener Zweiphasenstrom 110 wird einem Abscheider 10 zugeführt, von dessen Kopf der Rückführstrom 106 gasförmig entnommen wird, und aus dessen Sumpf ein Rohmethanolstrom 111 abgezogen wird.

Die weitere Behandlung des Rohmethanolstroms 111 wird hier nicht gesondert veranschaulicht und kann in jeder aus dem Stand der Technik bekannten und zweckmäßigen Weise erfolgen. Stellvertretend für unterschiedliche Ausgestaltungen ist eine Methanolaufbereitung schematisch mit 11 angeben. Insbesondere kann in einer derartigen Methanolaufbereitung 11 eine sogenannte Toppingkolonne und eine sogenannte Refiningkolonne vorgesehen sein. Aus der Methanolaufbereitung 11 werden unterschiedliche Stoffströme 112 abgezogen, die beispielsweise einen Methanolproduktstrom, einen Fuselölstrom, einen Wasserstrom und einen Strom mit leichteren Komponenten umfassen können.

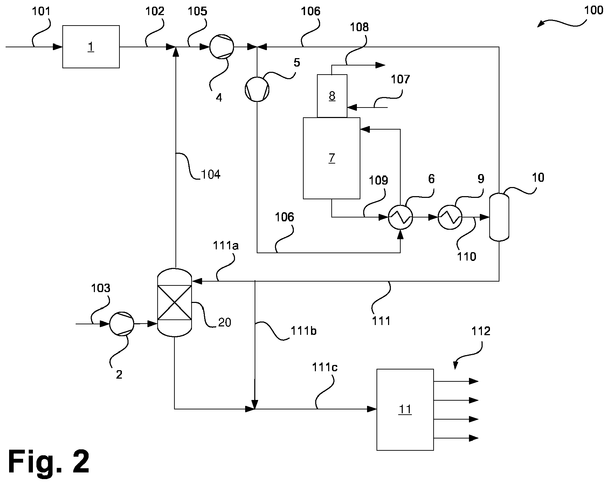

In Figur 2 ist ein Verfahren gemäß einer hier vorgeschlagenen Ausgestaltung veranschaulicht und insgesamt mit 100 bezeichnet.

Die Kohlendioxidreinigung, in Figur 1 mit 2 bezeichnet, wird hier unter Verwendung einer Rohmethanolwaschkolonne 20 durchgeführt, die mit einem Anteil 111a des Rohmethanolstroms 111 als Waschmittel betrieben wird. Weitere Aufreinigungsschritte können zusätzlich erfolgen, beispielsweise falls die Rohmethanolwäsche nicht ausreicht, um die geforderte Einsatzqualität darzustellen. Das mit absorbierten Komponenten beladene Waschmittel wird mit einem nicht als Waschmittel verwendeten Anteil 111b des Rohmethanolstroms 111 vereinigt und als Sammelstrom 111c der Methanolaufbereitung 11 zugeführt.

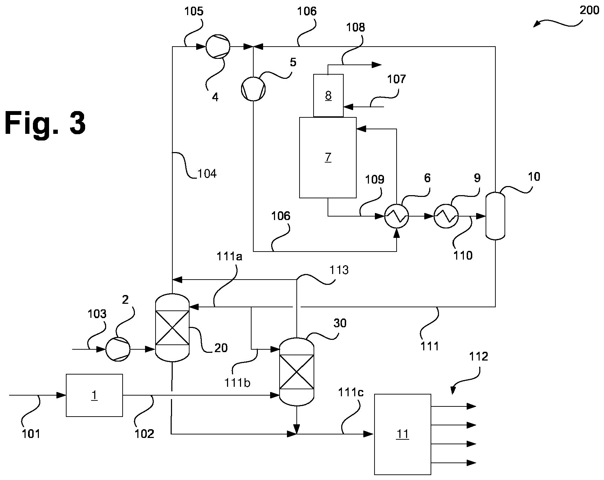

In Figur 3 ist ein Verfahren gemäß einer weiteren, hier vorgeschlagenen Ausgestaltung veranschaulicht und insgesamt mit 200 bezeichnet.

Verfahren 200 eignet sich insbesondere für Fälle, in denen nur ein Teil des Rohmethanols für die Entfernung von Katalysatorgiften aus dem Rohkohlendioxidstrom 103 erforderlich ist. Das verbleibende Rohmethanol kann in diesem Fall einem Wasserstoffstripper 30 zugeführt werden, der mit dem Wasserstoffstrom 102 als Strippgas betrieben werden kann. Auf diese Weise kann in dem Rohmethanolstrom 111 bzw. 111b enthaltenes Kohlendioxid zumindest teilweise in einen Kopfstrom 113 überführt werden, der in der veranschaulichten Weise zurückgeführt werden kann.

## Patentansprüche

**1.** Verfahren (100, 200) zur Herstellung von Methanol, das umfasst:
Reinigen eines Rohkohlendioxidstroms (103) unter Erhalt eines Reinkohlendioxidstroms (104);
Einspeisen des Reinkohlendioxidstroms (104) in einen Methanolsynthesekreislauf; und
Ausschleusen eines Rohmethanolstroms (111) aus dem Methanolsynthesekreislauf;
wobei das Reinigen des Rohkohlendioxidstroms (103) absorptiv und unter Verwendung des Rohmethanolstroms (111) oder eines Anteils (111a) hiervon als Absorptionsflüssigkeit vorgenommen wird.

**2.** Verfahren (100, 200) nach Anspruch 1, wobei
das Reinigen des Rohkohlendioxidstroms (103) auf einem Druck in einem Druckbereich von 5 bis 100 bar vorgenommen wird.

**3.** Verfahren (100, 200) nach Anspruch 1 oder 2, wobei
das Rohmethanol einen durch Methanol und Wasser gebildeten Anteil aufweist, der 45 bis 95 mol-% Methanol und im verbleibenden Rest Wasser umfasst.

**4.** Verfahren (100, 200) nach einem der vorstehenden Ansprüche, wobei
der Rohmethanolstrom (111) oder der für das Reinigen des Rohkohlendioxidstroms (103) verwendete Anteil (111a) des Rohmethanolstroms (111) nach seiner Verwendung für das Reinigen des Rohkohlendioxidstroms (103) einer Methanolaufbereitung (11) zugeführt wird.

**5.** Verfahren (100, 200) nach einem der vorstehenden Ansprüche, wobei
die Methanolaufbereitung unter Verwendung wenigstens zweier Aufreinigungskolonnen durchgeführt wird.

**6.** Verfahren (200) nach einem der vorstehenden Ansprüche, wobei
für das Reinigen des Rohkohlendioxidstroms (103) ein erster Anteil (111a) des Rohmethanolstroms (111) verwendet wird und unter Verwendung von Wasserstoff Kohlendioxid aus einem zweiten Anteil (111b) des Rohmethanolstroms (111) ausgetrieben wird, wobei das ausgetriebene Kohlendioxid oder ein Teil hiervon in den Methanolsynthesekreislauf zurückgeführt wird.

**7.** Verfahren (100, 200) nach einem der vorstehenden Ansprüche, wobei
der Methanolsynthesekreislauf einen Methanolsynthesereaktor (7) umfasst, dem ein Methanol, Wasser und Kohlendioxid enthaltender Produktstrom (109) entnommen wird, welcher unter Erhalt des Rohmethanolstroms (111) und eines Rückführstroms (106) einer Teilkondensation unterworfen wird.

**8.** Verfahren (100, 200) nach Anspruch 7, wobei
der Methanolsynthesereaktor (7) als Isothermalreaktor ausgebildet ist, der ein Katalysatorbett aufweist, in dem gewickelte Dampferzeugungsrohre angeordnet sind, wobei die Dampferzeugungsrohre mit Kesselspeisewasser beschickt werden, und wobei mittels der Dampferzeugungsrohre Dampf erzeugt wird.

**9.** Verfahren (100, 200) nach Anspruch 7 oder 8, wobei
der Rückführstrom (106) oder ein Teil hiervon mit Kohlendioxid des Reinkohlendioxidstroms (104) und Wasserstoff vereinigt, verdichtet, erwärmt und dem Methanolsynthesereaktor (7) erneut zugeführt wird.

**10.** Verfahren (100, 200) nach Anspruch 7 oder 8, wobei
der Methanolsynthesereaktor (7) unter Verwendung eines Kupfer, Zinkoxid und Aluminiumoxid enthaltenden Katalysators betrieben wird.

**10.** Verfahren (100, 200) nach einem der vorstehenden Ansprüche, wobei
das Reinigen des Rohkohlendioxidstroms (103) derart durchgeführt wird, dass ein Gehalt des Reinkohlendioxidstroms (104) an zumindest einer schwefelhaltigen Verbindung und/oder zumindest einer stickstoffhaltigen Verbindung und/oder zumindest einer Halogenverbindung und/oder von Cyanwasserstoff unterhalb eines vorgegebenen Schwellwerts liegt.

**11.** Verfahren (100, 200) nach einem der vorstehenden Ansprüche, bei dem sauerstoffhaltiger Rohwasserstoff (101) unter Verwendung einer katalytischen Reinigungseinheit (1) einer Sauerstoffentfernung unterworfen wird.

**12.** Anlage zur Herstellung von Methanol, die zur Durchführung der folgenden Schritte eingerichtet ist:
Reinigen eines Rohkohlendioxidstroms (103) unter Erhalt eines Reinkohlendioxidstroms (104);
Einspeisen des Reinkohlendioxidstroms (104) in einen Methanolsynthesekreislauf; und
Ausschleusen eines Rohmethanolstroms (111) aus dem Methanolsynthesekreislauf;
wobei das Reinigen des Rohkohlendioxidstroms (103) absorptiv und unter Verwendung des Rohmethanolstroms (111) oder eines Anteils (111a) hiervon als Absorptionsflüssigkeit vorgenommen wird.

**13.** Anlage nach Anspruch 12, die zur Durchführung eines Verfahrens nach einem der Ansprüche 1 bis 11 eingerichtet ist.
